# EUROPEAN PATENT APPLICATION

(11) **EP 4 166 679 A1**
(43) Date of publication of application: **19.04.2023**
(21) Application number: 21823030.8
(22) Date of filing: 10.06.2021
(51) Int. Cl.: C12Q 1/70, C12Q 1/6804

(54) **LINE ELIMINATION IMMUNOCHROMATOGRAPHIC TEST PAPER AND APPLICATION THEREOF IN CRISPR NUCLEIC ACID ASSAY**

(30) Priority: 12.06.2020 CN 202010533739
(71) Applicant: Academy of Military Medical Science, Academy of Military Science, People's Liberation Army of China, Beijing 100071 (CN)
(72) Inventor: LI, Hao, Beijing 100071 (CN); SUN, Yansong, Beijing 100071 (CN); WANG, Yanhe, Beijing 100071 (CN); DONG, Xue, Beijing 100071 (CN)
(74) Representative: Isarpatent
(86) International application number: PCT/CN2021/099306
(87) International publication number: WO 2021/249459

(57) **Abstract**

The invention discloses a "line elimination" immunochromatographic strip and its application in CRISPR nucleic acid detection. The invention provides a kit, including the following: 1) Immunochromatographic strip, the immunochromatographic strip includes a sample pad containing a colloidal gold-labeled antibody, an NC membrane containing a T line and a C line, and a absorbent pad in sequence in the flow direction of the sample; the T line is formed by streptavidin; the C line is formed by a secondary antibody of the colloidal gold-labeled antibody; 2) a CRISPR reaction system, the CRISPR reaction system includes a reporter RNA, a crRNA and a Cas protein; the Cas protein is the Cas protein in the class II type V or VI CRISPR system. The experiments of the invention have proved that by combining the CRISPR nucleic acid detection system, the strip in the kit can realize the detection of various specific nucleic acids (pathogens, genes mutations, drug resistance mutations, etc.) with high sensitivity, high specificity, and convenient detection.

## Description

### Technical field

The invention relates to a "line elimination" immunochromatographic strip and its application in CRISPR nucleic acid detection, and belongs to the technical field of molecular diagnosis.

### Background of the invention

In recent years, CRISPR gene-editing technology has triggered tremendous changes in the biological field. In 2017, the researchers discovered that *Leptotrichiawadei*-Cas13a protein (*Lw*Cas13a) can be used for nucleic acid detection, and by combining the *Lw-*Cas13a protein and recombinase polymerase amplification (RPA) technology, they established a highly sensitive and highly specific nucleic acid detection method, SHERLOCK (Specific High-sensitivity Enzymatic Reporter UnLOCKing). The sensitivity of this method has reached a single copy and the specificity has reached a single base, that means the sensitivity of the system is increased by 1 million times. The system has been used for the detection of Zika and Dengue virus in biological samples (blood or urine) as well as cell-free mutant tumor DNA in human body.

In 2018, the researchers established a CRISPR-Cas13a nucleic acid rapid detection technology suitable for lateral flow immunochromatographic assay (LFCA) by modifying the biotin and small molecule antigen FAM at both ends of reporter RNA. Compared with traditional methods, LFCA has these advantages, including simplicity, speed, portability, on-site detection and no need for professional operators. At present, the main principle of LFCA strip suitable for CRISPR nucleic acid detection is the "sandwich method". The specific detection principle is: both ends of the reporter RNA are respectively modified with biotin and FAM. For positive samples in the CRISPR reaction system, the double-labeled reporter RNA cleaved by the Cas protein and flowed from the side of the sample pad to the binding pad due to capillary force.

The FAM end of the reporter RNA will bind to colloidal gold particles, the other end will be captured by streptavidin (C line) in chromatography, and the sheared reporter RNA-FAM-colloidal gold particle complex will specifically bind to the secondary antibody on the T line, and accordingly, T line and C line develop color concurrently. However, in actual use, it is found that no matter the negative or positive samples there will be bands in both, in the detection of LFCA strip based on above principle, especially when the concentration of samples is low, although the color shades of the T line and C line in the negative and positive samples are different, which also brings certain difficulties to the interpretation of the results that mainly rely on subjective naked eyes observation.

Therefore, it is a research hotspot to find a new CRISPR nucleic acid detection method.

### Summary of The present invention

One object of the present invention is to provide a target nucleic acid detection kit. The kit provided by the invention comprises the following:

### 1) Immunochromatographic strip

The immunochromatographic strip comprises a sample pad containing colloidal gold-labeled antibody, a NC membrane containing the T line and C line, and an absorbent pad in sequence in the flow direction of the sample.

The T line is coated with streptavidin.

The C line is coated with a secondary antibody of the colloidal gold-labeled antibodies.

### 2) CRISPR reaction system.

The CRISPR reaction system includes a reporter RNA, a crRNA and a Cas protein.

The Cas protein is a Cas protein in the class II type V or VI CRISPR system.

The CRISPR-Cas13a reaction system is recommended to use in above CRISPR reaction system, but does not exclude the use of other CRISPR reaction systems, including the class II type V and VI CRISPR reaction system (Class II, type V or VI, for example CRISPR-Cas12a, CRISPR-Cas12b, CRISPR-Cas12b, CRISPR-Cas13b, etc.)

Both ends of the reporter RNA are respectively labeled with biotin and a group that can bind up with the colloidal gold-labeled antibody.

The crRNA includes the Cas protein binding region and the nucleic acid binding region of target sequence.

In the above kit, the Cas protein is the *Lw*Cas13a protein, Cas 12a or Cas 12b protein.

In the above kit, the reporter RNA consists of 20 U nucleotide, and both ends of the reporter RNA are respectively labeled with biotin and fluorescein, but other labeling methods, nucleotide types and quantities of reporter RNA are not excluded.

In the above kit, the kit further includes the amplification reagent for amplifying the target nucleic acid.

In the above kit, the amplification is RAA isothermal amplification, PCR variable temperature amplification or LAMP isothermal amplification, but other pre-amplification methods (such as PCR variable temperature amplification, LAMP isothermal amplification, etc.) and no pre-amplification detection are not excluded. In embodiment of the invention, the amplification reagent for amplifying the target nucleic acid is specifically an RAA isothermal amplification kit.

In the above kit, the target nucleic acid is a pathogenic nucleic acid, a nucleic acid with single nucleotide polymorphism or a drug resistant nucleic acid, etc.

In the above kit, the pathogenic nucleic acid is from severe acute respiratory syndrome coronavirus-2 (SARS-CoV-2), Ebola virus (EBOV) or forest encephalitis virus (FEV, or tick-borne encephalitis virus, TBEV). In an embodiment of the invention, the pathogenic nucleic acid is specifically SARS-CoV-2 nucleic acid, EBOV nucleic acid or TBEV nucleic acid.

In the above kit, the colloidal gold-labeled antibody is an anti-FITC antibody. In an embodiment of the invention, the colloidal gold-labeled antibody is the rabbit derived anti-FITC antibody, and the secondary antibody of the rabbit derived anti-FITC antibody is a goat anti-rabbit IgG.

In an embodiment of the invention, the components of the CRISPR reaction system are as follows: reporter RNA, crRNA, *Lw*Cas13a protein, NTP Mix, T7 RNA polymerase, RNase inhibitor, MgCl₂, HEPES buffer, and Nuclease-free water.

The coating concentration of the colloidal gold-labeled anti-FITC antibody is 0.016-0.16 mg/mL.

The coating concentration of streptavidin is 1.0-2.0 mg/mL.

The concentration of the reporter RNA contained in the CRISPR reaction system is 2-20 nM.

The concentration of the crRNA in the CRISPR reaction system is 5.5-55 nM.

The concentration of rNTP in the CRISPR reaction system is 1-10 mM.

The concentration of the RNase inhibitor in the CRISPR reaction system is 0.8-8 IU/µL.

The concentration of the Cas13a protein in the CRISPR reaction system is 22.5-225 nM.

The concentration of the T7 transcriptase in the CRISPR reaction system is 0.5-5 IU/µL.

The concentration of MgCl₂ in the CRISPR reaction system is 5-50 mM.

The concentration of HEPES in the CRISPR reaction system is 10-100 mM.

The volume of RT-RAA product in the CRISPR reaction system is 5-10 µL.

In the above kit:
If the target nucleic acid is the SARS-CoV-2 genome (GenBank ID: MN908947.3), the nucleotide sequence of the corresponding crRNA is that shown in SEQ ID NO: 2, and the primers used in the RT-RAA amplification kit to amplify the target nucleic acid are 2019-nCoV-F1 primer shown in SEQ ID NO: 3 and 2019-nCoV-F1 primer shown in SEQ ID NO: 4.

If the target nucleic acid is the EBOV genome (GenBank ID: AF086833.2), the nucleotide sequence of the corresponding crRNA is that shown in SEQ ID NO: 5, and the primers used in the RT-RAA amplification kit to amplify the target nucleic acid are EBOV-F1 primer shown in SEQ ID NO: 6 and EBOV-R1 primer shown in SEQ ID NO: 7.

If the target nucleic acid is the TBEV genome (GenBank ID: MH645618.1), the nucleotide sequence of the corresponding crRNA is that shown in SEQ ID NO: 8, and the primers used in the RT-RAA amplification kit to amplify the target nucleic acid are TBEV-F1 primer shown in SEQ ID NO: 9 and TBEV-R1 primer shown in SEQ ID NO: 10.

The use of the above kit in the preparation of products with at least one function in the following 1)-4) is also within the protection scope of the present invention:
1) Detect whether a target nucleic acid is a pathogenic nucleic acid;
2) Detect a SNP site polymorphism of the target nucleic acid;
3) Detect whether a target nucleic acid is a drug resistance gene;
4) Detect whether a sample to be tested contains a pathogenic nucleic acid.

In the above use, the nucleic acid of the pathogen is from the SARS-CoV-2, Ebola virus or forest encephalitis virus, but not limited thereto.

Another object of the invention is to provide the following method:
The invention provides a method for detecting whether a target nucleic acid is a pathogenic nucleic acid, comprising these following steps:
1) Amplify target nucleic acid using the reagent for RAA isothermally amplifying in the above kit to obtain the RT-RAA amplification product;
2) Add the RT-RAA amplification product to the CRISPR reaction system in the above kit to obtain a CRISPR reaction product;
3) Detect the CRISPR reaction product using the immunochromatographic strip in the above kit.

If the test paper has no T line and a C line appears, the target nucleic acid is the pathogenic nucleic acid or a candidate for the pathogenic nucleic acid, and it is judged to be positive. If the test paper has a T line and a C line, the target nucleic acid is not the target nucleic acid or a candidate for the target nucleic acid, and it is judged to be negative.

If, on the lateral flow strip, the C line was visible, while the T line was absent, the target nucleic acid is the pathogenic nucleic acid and a positive result was recorded, whereas if both the T line and C line were visible, the target nucleic acid is not the target nucleic acid and the result was recorded as negative.

The invention also provides a method for detecting whether a sample to be tested is a pathogenic nucleic acid, which comprises these following steps:
1) Amplify a sample to be tested using a reagent for RAA isothermal amplification in the above kit to obtain a RT-RAA amplification product;
2) Add the RT-RAA amplification product to the CRISPR reaction system in the above kit to obtain a CRISPR reaction product;
3) Detect the CRISPR reaction product using the immunochromatographic strip in the above kit.

If, on the lateral flow strip, the C line was visible, while the T line was absent, the sample to be tested contains the pathogenic nucleic acid or a candidate for the pathogenic nucleic acid, and a positive result was recorded, whereas if both the T line and C line were visible, the sample to be tested does not contain the target nucleic acid or a candidate for the target nucleic acid and the result was recorded as negative.

In the above methods, the optimum reaction temperature for CRISPR detection is 37°C, the optimum reaction time for CRISPR detection is 30 min, and the optimum read out time for this test strip is 2 min.

In the above method, the nucleic acid of the pathogen is from SARS-CoV-2, Ebola virus or forest encephalitis virus, but not limited thereto.

The invention will be further described in conjunction with embodiments.

### Brief Description of the Drawings

Fig. 1 shows the schematic diagram of the "line elimination" lateral flow immunochromatographic strip.
Fig. 2 shows a comparison of the sensitivity test of the "line elimination" CRISPR lateral flow strip with the existing CRISPR nucleic acid detection strip.
Fig. 3 shows the specificity test results of the "line elimination" CRISPR lateral flow strip.
Fig. 4 shows CRISPR detection results of other pathogens using the "line elimination" CRISPR lateral flow strip.
Fig. 5 shows detection results of different concentrations of colloidal gold-labeled rabbit derived anti-FITC antibodies.
Fig. 6 shows detection results of ERASE strip at different coating concentrations of streptavidin.
Fig. 7 shows detection results of different classes of reporter RNA.
Fig. 8 shows detection results of different concentrations of reporter RNA.
Fig. 9 shows detection results of different concentrations of crRNA.
Fig. 10 shows detection results of different concentrations of rNTP.
Fig. 11 shows detection results of different concentrations of RNase inhibitors.
Fig. 12 shows detection results of different concentrations of Cas13a proteins.
Fig. 13 shows detection results of different concentrations of T7 transcriptase.
Fig. 14 shows detection results of different concentrations of MgCl₂.
Fig. 15 shows detection results of different concentrations of HEPES.
Fig. 16 shows detection results of CRISPR "line elimination" strip with different nucleic acid loading amounts.
Fig. 17 shows detection results of the "line elimination" strip at different reaction temperatures for CRISPR detection.
Fig. 18 shows detection results of the "line elimination" strip under different reaction times for CRISPR detection.
Fig. 19 shows detection results of the "line elimination" strip at different result read out times.

### Best Mode of Implementing The present invention

The experimental methods used in the following examples are conventional methods unless otherwise specified.

The materials and reagents used in the following examples can be obtained from commercial sources unless otherwise specified.

The following examples are provided so as to facilitate a better understanding of the present invention, but not to limit the present invention.

Quantitative experiments in the following examples are all set up to repeat three times, and the results are averaged.

The sources of reagents in the following examples are as follows: NTP mix (NEB, N0466S, 25 mM), RNase inhibitor (Murine RNase inhibitor, NEB, M0314L), T7 RNA polymerase (NEB, M0251S, 5000 U/ml), RT-RAAAmplification Kit (Hangzhou Zhongce, S003ZC, 40000 U/ml), Rabbit Anti-FITC Antibody (Shanghai Shenggong, D110003), Streptavidin (Suolaibao, S9170-10 mg), Goat Anti-Rabbit IgG (Thermo Fisher, N24916), Proclin300 (sigma, 48914-U), nitrocellulose membrane (Sartorius, CN140), glass fiber (Shanghai Liangxin, CB08), polyester fiber (Shanghai Liangxin, DL42), absorbent paper (Shanghai Liangxin, CH37K), HEPES buffer (Suolaibao, YZ-B-HEPES250), MgCl₂ solution (Tiangen Biochemical, RP107), existing CRISPR lateral flow strip (TwistDx, MileniaHybridetect 1), LwCas13a protein (Ningbo Jiangshen, DB005), SARS-CoV-2 Nucleic Acid Reference Material (National Institute of Metrology, GBW(E)091089).

The nucleotide sequences used in the following examples are as follows:
SEQ ID NO: 1 shows the sequence of the reporter RNA (20U);
SEQ ID NO: 2 shows the sequence of 2019-nCoV-crRNA;
SEQ ID NO: 3 shows the sequence of 2019-nCoV-F1 primer;
SEQ ID NO: 4 shows the sequence of 2019-nCoV-R1 primer;
SEQ ID NO: 5 shows the sequence of EBOV-crRNA;
SEQ ID NO: 6 shows the sequence of EBOV-F1 primer;
SEQ ID NO: 7 shows the sequence of EBOV-R1 primer;
SEQ ID NO: 8 shows the sequence of TBEV-crRNA;
SEQ ID NO: 9 shows the sequence of TBEV-F1 primer;
SEQ ID NO: 10 shows the sequence of TBEV-R1 primer;
SEQ ID NO: 11 shows the sequence of 2019-nCoV-F-N primer; and
SEQ ID NO: 12 shows the sequence of 2019-nCoV-R-N primer.

### Example 1. Nucleic acid detection kit based on CRISPR detection principle and "line elimination" immunochromatography

### I. Preparation of the nucleic acid detection kit based on CRISPR detection and "line elimination" immunochromatography

### 1. Preparation of "line elimination" immunochromatographic strip

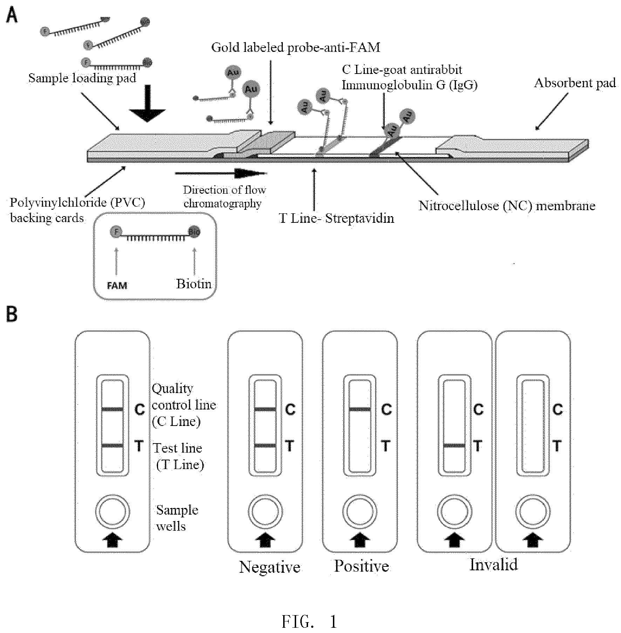

The lateral flow strip based on the "line elimination" method (as shown in Fig. 1A) includes three components: the sample pad containing the colloidal gold-labeled rabbit derived anti-FITC antibody, the NC membrane containing a T line and a C line, and a absorbent pad, and the sequence is arranged in the direction of flow.

### 1) Sample pad containing a colloidal gold-labeled rabbit derived anti-FITC antibody

Rabbit derived FITC antibody is purchased from Shanghai Shenggong, product number: D110003

Colloidal gold solution: add 0.4 mL (10%, mass volume ratio g:mL) of chloroauric acid and 0.4 mL (10%, mass volume ratio g:mL) trisodium citrate per 100 mL purified water to obtain a colloidal gold solution.

Colloidal gold-labeled rabbit derived anti-FITC antibody solution: add 0.005 mL (0.2 M) potassium carbonate aqueous solution and 0.01 mg rabbit anti-FITC antibody to per 1 mL of the above colloidal gold solution to obtain a colloidal gold-labeled rabbit derived anti-FITC antibody solution with a concentration of 0.01 mg/mL.

Spray 1 mL colloidal gold-labeled rabbit derived anti-FITC antibody solution on a sample pad with an area of 0.006 m², and the coating concentration is 0.02 mg/mL to obtain a sample pad containing the colloidal gold-labeled rabbit derived anti-FITC antibody.

### 2) Preparation of NC membranes containing a T line and a C line

Streptavidin solution, the solute is streptavidin (Suolaibao, S9170-10 mg), the solvent is phosphate coating buffer (each 100 mL contains 0.8 g NaCl, 0.58 g Na₂HPO₄·12H₂O, 0.06 g NaH₂PO₄·2H₂O, 0.02 mLProclin300 (sigma, 48914-U), and dilute to 100 mL with purified water), the concentration of buffer is 1.5 mg/mL.

Goat anti-rabbit IgG (a secondary antibody of anti-FITC antibody) solution, the solute is a goat anti-rabbit IgG (Thermo Fisher, N24916), the solvent is phosphate coating buffer, its concentration is 1 mg/mL.

Spray 30 µL of the above streptavidin solution on the NC membrane to form a T line with a coating concentration of 1.5 mg/mL; spray 30 µL of the goat anti-rabbit IgG solution on the nitrocellulose membrane (NC membrane) to form a C line, the coating concentration is 1 mg/mL; and the distance between the T line and the C line is 7 mm, to obtain an NC membrane containing a T line and a C line.

### 3) Assembly of "line elimination" immunochromatographic strip

The above-mentioned sample pad containing the colloidal gold-labeled rabbit derived anti-FITC antibody, the NC membrane containing a T line and a C line, and the absorbent pad are sequentially assembled on the PVC backing cards in the chromatographic direction to obtain the "line elimination" immunochromatographic strip.

### 2. CRISPR reaction system

The reaction system includes the following substances:

### 1) Reporter RNA

Custom and synthesized from Beijing Tianyi Huiyuan Biotechnology Co., Ltd.

The reporter RNA (20U) consists of 20 U nucleotide, the nucleotide sequence is shown in SEQ ID NO: 1, and both ends of the RNA are respectively labeled with FAM and biotin, wherein FAM binds to the colloidal gold-labeled rabbit derived anti-FITC antibody, and biotin binds to the T line streptavidin on strips.

### 2) Preparation of crRNA

crRNA consists of a binding region of *Lw*Cas13a protein and a sequence binding region of target nucleic acid.

### 3) LwCas13a protein, NTP Mix, T7 RNApolymerase, RNase inhibitor, MgCl₂ solution and HEPES buffer.

### 3. Preparation of the nucleic acid detection kit based on CRISPR detection principle

The "line elimination" lateral flow immunochromatography strip prepared above and the above CRISPR reaction system are packaged separately to construct a nucleic acid detection kit based on the CRISPR detection principle (based on the CRISPR detection principle and the "line elimination" immunochromatography nucleic acid detection kit).

### II. Detection principle and establishment of method of nucleic acid detection kit based on CRISPR detection principle and "line elimination" immunochromatography

### 1. Detection principle of nucleic acid detection kit based on CRISPR detection principle and "line elimination" immunochromatography

If the sample to be tested contains the target nucleic acid, the target nucleic acid binds to the crRNA in the CRISPR reaction system to activate the activity of *Lw*Cas13a protein in the CRISPR reaction system, the reporter RNA bound to the FAM group and biotin at the end thereof in CRISPR detection system will be cleaved by *Lw*Cas13a protein. The obtained reaction system binds to the colloidal gold-labeled rabbit derived anti-FITC antibody when passing through the sample pad of the "line elimination" immunochromatographic strip, and a system containing the colloidal gold-labeled rabbit derived anti-FITC antibody-FAM group is obtained. When passing through the T line, it does not bind to streptavidin, and the T line does not develop color. When it reaches the C line, it is captured by the C line antibody (goat anti-rabbit IgG-colloidal gold-labeled rabbit derived anti-FITC antibody-FAM group) and the captured colloid gold precipitates so as to develop color, C line develops color (only one quality control line appears on the strip).

If the sample to be tested does not contain the target nucleic acid, and the target nucleic acid cannot activate the activity of *Lw*Cas13a protein in the CRISPR reaction system, the reporter RNA whose terminal is bound to the FAM group and biotin in the CRISPR detection system will not be cleaved by *Lw*Cas13a protein, and when passing through the sample pad of "line elimination" immunochromatographic strip, the obtained reaction system binds to the colloidal gold-labeled rabbit derived anti-FITC antibody to obtain a system containing colloidal gold-labeled rabbit derived anti-FITC antibody-FAM group-biotin, and when passing through the T line, the colloidal gold-labeled rabbit derived anti-FITC antibody-FAM group-biotin on the biotin binds to streptavidin on the T line, the colloidal gold precipitates on the T line to develop color, and when it passes through the C line, the rabbit source anti-FITC antibody on the colloidal gold-labeled rabbit source anti-FITC antibody-FAM group-biotin and captured colloidal gold goat anti-rabbit IgG at the C line precipitate to develop color (goat anti-rabbit IgG-colloidal gold-labeled rabbit anti-FITC antibody-FAM group -biotin), C line develops color (two lines appear on the strip, which are the quality control line and the detection line respectively).

### 2. Establishment of Method

### 1) Amplification of target nucleic acids

The nucleic acid in the sample to be tested is extracted as the target nucleic acid, which is used as a template for pre-amplification.

The RT-RAA amplification kit is used to pre-amplify the target nucleic acid. The brief steps are as follows: firstly, prepare a mixture of 2 µL template, 2 µL upstream and downstream primers (10 µM), and 41.5 µL A Buffer, and add it to a basic reaction unit containing lyophilized powder. In the basic reaction unit, the lyophilized powder is fully redissolved evenly. Add 2.5 µL B Buffer solution to the cap of each reaction tube, close the cap, invert it up and down 5-6 times to mix well, and centrifuge at a high speed for 10 seconds. The above reaction tube is placed at 42°C for 30 min to react to obtain a RT-RAA amplification product.

### 2) CRISPR reaction

Use *Lw*Cas 13a protein and reporter RNA (20U) for CRISPR reaction, the components in 50µL volume CRISPR reaction system are as follows:
5 µL RT-RAAproduct; 2 µL LwCas13a protein (1.13 µM, Ningbo Jiangshen, DB005); 4 µL NTP Mix (NEB, N0466S); 1 µL T7 RNA polymerase (NEB, M0251S); 2 µL RNase inhibitor (Murine RNase inhibitor, NEB, M0314L); 0.5 µL MgCl₂ solution (1 M); 1 µL HEPES buffer (1 M, Suolaibao, YZ-B-HEPES250); 3 µL corresponding crRNA (375 nM); 5 µL reporter RNA (20 nM); 26.5 µL enzyme-free water.

Cover the reaction tube of the above reaction system with a cap, invert it up and down 5-6 times to mix well, centrifuge at a low speed for 10 seconds, and place it at 37°C for 30 min to react to obtain a CRISPR reaction product.

### 3) Detection using strips

Add all the CRISPR reaction products to the sample wells of the "line elimination" immunochromatography strip of the nucleic acid detection kit based on the CRISPR detection principle, let it stand for 2-5 min to observe with the naked eyes and take pictures to record the results.

Negative control: prepare a CRISPR system that does not contain a RAA amplification product, and add all of them to the sample wells of the "line elimination" immunochromatography strip, let it stand for 2-5 min to observe with the naked eyes and take pictures to record the results.

Judgment criteria are as follows (Fig. 1B): If the strip has no T line and a C line appears, the sample to be tested contains the target nucleic acid or a candidate for the target nucleic acid, and it is judged to be positive. If the strip has a T line and a C line, the sample to be tested does not contain the target nucleic acid or a candidate for the target nucleic acid, and it is judged to be negative. If no C line appears on the strip, the strip is invalid, and the strip needs to be replaced and tested again.

### Example 2. Comparison of the nucleic acid detection kit based on CRISPR detection principle and "line elimination" immunochromatography with the existing CRISPR lateral flow chromatography strip in the detection of SARS-CoV-2 nucleic acid

### I. Preparation of the nucleic acid detection kit based on CRISPR detection principle and "line elimination" immunochromatography

### 1. Preparation of "line elimination" immunochromatographic strip

The preparation is same as 1 of Example 1;

### 2. CRISPR reaction system

### 1) Obtaining of the reporter RNA

The obtaining is the same as that in 2 of Example 1.

### 2) Preparation of crRNA

crRNA consists of a binding region of *Lw*Cas13a protein and a sequence binding of target nucleic acid.

The nucleotide sequence of the crRNA is shown in SEQ ID NO: 2, wherein positions 1-38 in SEQ ID NO: 2 are the region binding to *Lw*Cas13a protein, and positions 39-66 are the region binding to the target sequence in the target nucleic acid (SARS-CoV-2 nucleic acid).

### 3) LwCas13a protein, NTP Mix, T7 RNApolymerase, RNase inhibitor, MgCl₂ solution and HEPES buffer.

### 3. Preparation of the nucleic acid detection kit based on CRISPR detection principle

The "line elimination" immunochromatography strip prepared above and the above CRISPR reaction system are packaged separately to construct a nucleic acid detection kit based on the CRISPR detection principle (nucleic acid detection kit based on the CRISPR detection principle and "line elimination" immunochromatography).

### II. Detection using the nucleic acid detection kit based on CRISPR detection principle and "line elimination" immunochromatography

### 1) Amplification of a target nucleic acid

The target sequence is positions 29265-29292 of the SARS-CoV-2 Genome (GenBank ID: MN908947.3, March 18, 2020), and this target sequence is used as the standardized SARS-CoV-2 nucleic acid.

The standardized SARS-CoV-2 nucleic acids diluted with ddH₂O purified water to 10⁵, 10⁴, 10³, 10², 10¹, and 10⁰ copies/µL respectively are used as the target nucleic acids, and a negative control group (H₂O) is prepared.

The RT-RAA amplification kit is used to pre-amplify the target nucleic acid. The brief steps are as follows: firstly, 2 µL template, 2 µL upstream and downstream primers (10 µM, 2019-nCoV-F1 primer shown in SEQ ID NO: 3 and 2019-nCoV-F1 primer shown in SEQ ID NO: 4), 41.5 µL A Buffer to prepare a mixed solution, and add it to the basic reaction unit containing lyophilized powder, so that the lyophilized powder is fully redissolved evenly. Add 2.5 µL B Buffer solution to the cap of each reaction tube, close the cap, invert it up and down 5-6 times to mix well, and centrifuge at a high speed for 10 seconds. The above reaction tube is placed at 42°C for 30 min to react to obtain a RT-RAA amplification product.

### 2) CRISPR reaction

LwCas13a protein and the reporter RNA (20U) are used for CRISPR reaction, and components of the CRISPR reaction system are as follows:
5 µL RT-RAA product; 2 µL LwCas13a protein (1.13 µM, Ningbo Jiangshen, DB005); 4 µENTP Mix (NEB, N0466S); 1 µL T7 RNA polymerase (NEB, M0251S); 2 µL RNase inhibitor (NEB, M0314L)); 0.5 µL MgCl₂ solution (1 M); 1 µL HEPES buffer (1 M, Suolaibao, YZ-B-HEPES250)); 3 µL corresponding crRNA (375 nM); 5 µL reporter RNA (20nM); 26.5 µL enzyme-free water.

Cover the reaction tube of the above reaction system with a cap, invert it up and down 5-6 times to mix well, centrifuge at a low speed for 10 seconds, and place it at 37°C for 30 min to react to obtain a CRISPR reaction product.

### 3) Detection using strip

Add all the CRISPR reaction products to the sample wells of the "line elimination" immunochromatography strip of the nucleic acid detection kit based on the CRISPR detection principle, let it stand for 2-5 min to observe with the naked eyes and take pictures to record the results.

Negative control: Prepare a CRISPR reaction system that does not contain RT-RAA amplification products, and add all of them to the sample wells of the "line elimination" immunochromatography strip, let it stand for 2-5 min to observe with the naked eyes and take pictures to record the results.

Judgment criteria are as follows:
If the strip has no T line and a C line appears, the sample to be tested contains the target nucleic acid or a candidate for the target nucleic acid, and it is judged to be positive. If the strip has a T line and a C line, the sample to be tested does not contain the target nucleic acid or a candidate for the target nucleic acid, and it is judged to be negative. If no C line appears on the strip, the strip is invalid, and the strip needs to be replaced and tested again.

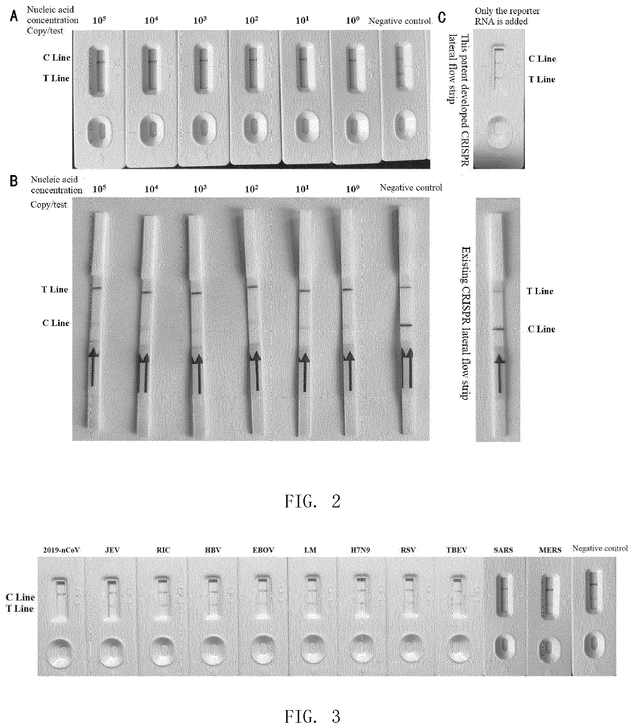

The results are shown in Fig. 2A. It can be seen that, except for the negative control group, where the C line and the T line appeared simultaneously (judged to be negative), the rest of groups showed only the C line (judged to be positive).

### III. Control: the sensitivity of the existing CRISPR lateral flow strip

Use the existing CRISPR lateral flow strip (TwistDx, MileniaHybridetect 1) instead of the above strip to perform step 3), and the rest of steps are the same as those for the "line elimination" CRISPR nucleic acid detection kit.

Judgment criteria are as follows:
If the strip has a T line and a C line, the sample to be tested contains the target nucleic acid or a candidate for the target nucleic acid, and it is judged to be positive. If the strip has no T line and a C line appears, the sample to be tested does not contain the target nucleic acid or a candidate for the target nucleic acid, and it is judged to be negative. If no C line appears on the strip, the strip is invalid, and the strip needs to be replaced and tested again.

The results are shown in Fig. 2B. It can be seen that, except for the negative control group where the C line and the T line appeared simultaneously (judged to be negative), the rest of groups showed weakened T lines and C lines (unable to judge).

### IV. Detection using the reaction system based on CRISPR detection principle and the "line elimination" immunochromatographic nucleic acid detection kit

Add the CRISPR reaction system (negative control) that does not contain RT-RAA amplification products to the sample wells of the "line elimination" immunochromatographic strip based on the CRISPR detection principle and the existing CRISPR lateral flow strip of the nucleic acid detection kit after the reaction is completed, let it stand for 2-5 min to observe with naked eyes and take pictures to record the results.

The results are shown in Fig. 2C. The C line and the T line appeared simultaneously in the "line elimination" immunochromatographic strip (this strip) (judged to be negative), and the C line and the T line appeared simultaneously in the existing CRISPR lateral flow strip (judged to be positive).

Therefore, it can be seen from the above that the sensitivity of the "line elimination" strip to the SARS-CoV-2 is single copy (10⁰ copies/µL), and it is easy to accurately interpret the experimental results. The degradation of a small amount of reporter RNA in the natural state will not affect the interpretation of the results. There are many subjective factors in the interpretation of existing CRISPR test strip results, and it is easy to have disagreements on whether there are C lines and T lines. The degradation of a small amount of reporter RNA in the natural state seriously affects the interpretation of the results. To sum up, the lateral flow strip by the "line elimination" is more standardized in the interpretation of results.

### Example 3. The critical concentration repeatability of the nucleic acid detection kit based on the CRISPR detection principle and the "line elimination" immunochromatography in the detection of SARS-CoV-2 nucleic acids

### I. Preparation of the nucleic acid detection kit based on CRISPR detection principle and "line elimination" immunochromatography

The preparation is same as that in Example 2.

### II. Detection using the nucleic acid detection kit based on CRISPR detection principle and "line elimination" immunochromatography

### 1) Amplification of target nucleic acids

According to Example 2, the sensitivity of the "line elimination" strip for the SARS-CoV-2 is 10⁰ copies/µL, and 10⁰ copies/µL of the standardized SARS-CoV-2 nucleic acid is used as the target nucleic acid, and a negative control group (H₂O) is prepared.

The method is the same as that in II of Example 2.

### 2) CRISPR reaction

The method is the same as that in II of Example 2.

### 3) Detection using strips

The method is the same as that in II of Example 2.

20 repeated detections are carried out using the "line elimination" immunochromatographic strip of the nucleic acid detection kit based on the CRISPR detection principle.

The results are as follows: except for the negative control group where the C line and the T line appeared simultaneously (judged to be negative), the test groups showed only the C line (judged to be positive).

It shows that this kit has a highly repeatability of the critical concentration of the SARS-CoV-2 and can be detected stably.

### Example 4. The specificity of the nucleic acid detection kit based on the CRISPR detection principle and the "line elimination" immunochromatography in the detection of SARS-CoV-2 nucleic acids

### 1. Preparation of the nucleic acid detection kit based on CRISPR detection principle and the "line elimination" immunochromatography

The preparation is the same as that in I of Example 2.

### 2. Detection using the nucleic acid detection kit based on CRISPR detection principle and the "line elimination" immunochromatography

### 1) Amplification of target nucleic acids

Japanese encephalitis virus (JEV), rickettsia (RIC), hepatitis B virus (HBV), Ebola virus (EBOV), Listeria monocytogenes (LM), H7N9 influenza virus (H7N9), Respiratory Syncytial Virus (RSV), Forest Encephalitis Virus (FEV, or TBEV), SARS Virus (SARS), Middle East Respiratory Syndrome Coronavirus (MERS) pathogenic nucleic acids stored in our Lab are used as the templates for the experimental groups, and a negative control group (H₂O) and positive control group (2019-nCoV, GenBank ID: MN908947.3, March 18, 2020) are prepared.

The method is the same as in II of Example 2.

### 2) CRISPR reaction

The method is the same as in II of Example 2.

### 3) Detection using strips

The method is the same as in II of Example 2.

The results are shown in Fig. 3. Except for the positive control group (2019-nCoV) where only the C line appears (judged to be positive), the rest of groups showed the C line and the T line simultaneously (judged to be negative).

The above results show that this kit has a highly specificity for the SARS-CoV-2, and does not cross-reactions with other pathogens, especially similar pathogens such as SARS.

### Example 5. Use of the nucleic acid detection kit based on CRISPR detection principle and the "line elimination" immunochromatography in clinical detection of SARS-CoV-2

This clinical trial is carried out in accordance with the "Measures for the Administration of Registration of In Vitro Diagnostic Reagents" (State Food and Drug Administration Order No.5), "Technical Guidelines for Clinical Trials of In Vitro Diagnostic Reagents" (State Food and Drug Administration Announcement No. 16, 2014) According to the requirements of Hubei Provincial Center for Disease Control and Prevention, Liyuan Hospital Affiliated to Tongji Medical College of Huazhong University of Science and Technology, and Ningbo Huamei Hospital of University of Chinese Academy of Sciences, the three clinical trial institutions are approved by the ethics committees of their respective clinical trial institutions.

A total of 649 effective cases are received in this clinical trial, from Hubei Provincial Center for Disease Control and Prevention (347 cases), Liyuan Hospital Affiliated to Tongji Medical College of Huazhong University of Science and Technology (119 cases), Ningbo Huamei Hospital of University of Chinese Academy of Sciences (103 cases), Beijing Three clinical trial institutions including the Municipal Center for Disease Control and Prevention (80 cases), clinical case samples include freshly collected samples, frozen samples, nucleic acid (RNA) extracts from frozen samples from "suspected cases", "suspected clustered case patients", cases released from the quarantine, and people who meet discharge criteria.

This clinical trial adopts the method of comparative analysis, and the comparative reagent is the SARS-CoV-2 2019-nCoV nucleic acid detection kit (fluorescent PCR method) produced by Daan Gene Co., Ltd. of ZhongShan University.

### 1. Preparation of the nucleic acid detection kit based on CRISPR detection principle and the "line elimination" immunochromatography

The preparation is the same as that in I of Example 2.

### 2. Detection using the nucleic acid detection kit based on CRISPR detection principle and the "line elimination" immunochromatography

### 1) Amplification of target nucleic acids

The method is the same as that in II of Example 2.

### 2) CRISPR reaction

The method is the same as that in II of Example 2.

### 3) Detection using strips

The method is the same as that in II of Example 2.

Comparative reagents: the same sample is tested, with the SARS-CoV-2 2019-nCoV nucleic acid detection kit used as a control.

Statistical analysis is performed on 649 samples, and the results are shown in Table 1. The kit of the present invention is compared with the comparative reagent, and the positive coincidence rate of the results is 90.67% (243/268), and its 95% confidence interval is 86.59%-93.60%. The negative coincidence rate is 99.21% (378/381) and its 95% confidence interval is 97.71%-99.73%, the overall coincidence rate is 95.69% (621/649) and its 95% confidence interval is 93.84%-97.00%, and the Kappa value is 0.910, greater than 0.75, the hypothesis test is carried out on the Kappa value, and the result P is less than 0.05, indicating that the Kappa value is statistically significant. (Table 1).

**Table 1 is a 2×2 contingency table of clinical trial results**

| Detection results of strips | Detection results of comparative kit | | total |
|---|---|---|---|
| | Positive | Negative | |
| Positive | 243 | 3 | 246 |
| Negative | 25 | 378 | 403 |
| total | 268 | 381 | 649 |
| Positive coincidence rate | 90.67% (86.59%-93.60%) | | |
| Negative coincidence rate | 99.21% (97.71%-99.73%) | | |
| Total coincidence rate | 95.69% (93.84%-97.00%) | | |
| Kappa | 0.910(P=0.00) | | |

The above results show that the diagnostic results of the kit of the present invention are consistent with those of the current commonly used clinical diagnostic kits.

### Example 6. Use of the nucleic acid detection kit based on CRISPR detection principle and the "line elimination" immunochromatography in detection of other pathogens

Dilute standardized Ebola virus (EBOV) and forest encephalitis virus (TBEV) nucleic acids to 10⁵, 10⁴, 10³, 10², 10¹, 10⁰ copies/µL respectively, and a negative control group (H₂O) is prepared, detected by the "line elimination" lateral flow strip.

The target sequence used for Ebola virus (EBOV) is positions 1001-1028 in sequence of its genome (GenBank ID: AF086833.2, February 13, 2012).

The target sequence used for forest encephalitis virus (TBEV) is positions 11023-11050 in sequence of its genome (GenBank ID: MH645618.1, January 07, 2020).

### I. Preparation of the nucleic acid detection kit based on CRISPR detection principle and "line elimination" immunochromatography

### 1. Preparation of the "line elimination" immunochromatographic strip

The preparation is the same as that in 1 of Example 1.

### 2. CRISPR reaction system

### 1) Obtaining of the reporter RNA

The obtaining is the same as that in 2 of Example 1.

### 2) Preparation of crRNA

crRNA (EBOV) consists of a region binding to LwCas13a protein and a region binding to a target nucleic acid in a target sequence (EBOV target sequence), and its nucleotide sequence is shown in SEQ ID NO: 5, wherein positions 1-38 in SEQ ID NO: 5 are the region binding to LwCas13a protein, and positions 39-66 are the region binding to the target nucleic acid in the target sequence.

crRNA (TBEV) consists of a region binding to LwCas13a protein and a region binding to a target nucleic acid in a target sequence (TBEV target sequence), and its nucleotide sequence is shown in SEQ ID NO: 8, wherein positions 1-38 in SEQ ID NO: 8 are the region binding to LwCas13a protein, positions 39-66 are the region binding to the target nucleic acid in the target sequence.

### 3) LwCas13a protein, NTP Mix, T7 RNA polymerase, RNase inhibitor, MgCl₂ solution and HEPES buffer.

### 3. Preparation of the nucleic acid detection kit based on CRISPR detection principle

The "line elimination" immunochromatographic strip prepared above and the CRISPR reaction system above are packaged separately to construct a nucleic acid detection kit based on the CRISPR detection principle.

### II. Detection using the nucleic acid detection kit based on CRISPR detection principle and the "line elimination" immunochromatography

### 1) Amplification of target nucleic acids

Dilute Ebola virus (EBOV) nucleic acid (AF086833.2) and forest encephalitis virus (TBEV) nucleic acid (MH645618.1) with ddH₂O purified water to 10⁵, 10⁴, 10³, 10², 10¹, 10⁰ copies/µL respectively as target nucleic acids, and a negative control group (H₂O) is prepared.

The RT-RAA amplification kit is used to pre-amplify the target nucleic acids. The brief steps are as follows: firstly, prepare a mixture of 2 µL template, 2 µL upstream and downstream primers (10 µM), and 41.5 µL A Buffer, and add it to a container containing lyophilized powder. In the basic reaction unit, the lyophilized powder is fully redissolved evenly. Add 2.5 µL B Buffer solution to the cap of each reaction tube, close the cap, invert it up and down 5-6 times to mix well, and centrifuge at a high speed for 10 seconds. The above reaction tube is placed at 42°C for 30 min to react to obtain a RT-RAA amplification product.

The upstream and downstream primers in EBOV amplification are the EBOV-F1 primer shown in SEQ ID NO: 6 and the EBOV-R1 primer shown in SEQ ID NO: 7 respectively.

The upstream and downstream primers in TBEV amplification are the TBEV-F1 primer shown in SEQ ID NO: 9 and the TBEV-R1 primer shown in SEQ ID NO: 10 respectively.

### 2) CRISPR reaction

LwCas13a protein and the reporter RNA (20U) are used for CRISPR reaction, and the components of system are as follows:
5 µL RT-RAA product; 2 µL LwCas13a protein (1.13 µM, Ningbo Jiangshen, DB005); 4 µENTP Mix (NEB, N0466S); 1 µL T7 RNA polymerase (NEB, M0251S); 2 µL RNase inhibitor (NEB, M0314L)); 0.5 µL MgCl₂ solution (1 M); 1 µL HEPES buffer (1 M, Suolaibao, YZ-B-HEPES250); 3 µL corresponding crRNA (375 nM); 5 µL reporter RNA (20 nM); 26.5 µL enzyme-free water.

Cover the reaction tube of the above reaction system with a cap, invert it up and down 5-6 times to mix well, centrifuge at a low speed for 10 seconds, and place it at 37°C for 30 min to react to obtain a CRISPR reaction product.

### 3) Detection using strips

Add all the CRISPR reaction products to the sample wells of the "line elimination" immunochromatography strip of the nucleic acid detection kit based on the CRISPR detection principle, let it stand for 2-5 min to observe with the naked eyes and take pictures to record the results.

Negative control: Prepare a CRISPR reaction system that does not contain RT-RAA amplification products, and add all of them to the sample wells of the "line elimination" immunochromatography strip of the nucleic acid detection kit based on the CRISPR detection principle, and let it stand for 2-5 min, and observe with naked eyes and take pictures to record the results.

Judgment criteria are as follows:
If the strip has no T line and a C line appears, the sample to be tested contains the target nucleic acid or a candidate for the target nucleic acid, and it is judged to be positive. If the strip has a T line and a C line, the sample to be tested does not contain the target nucleic acid or a candidate for the target nucleic acid is judged to be negative. If no C line appears on the strip, the strip is invalid, and the strip needs to be replaced and tested again.

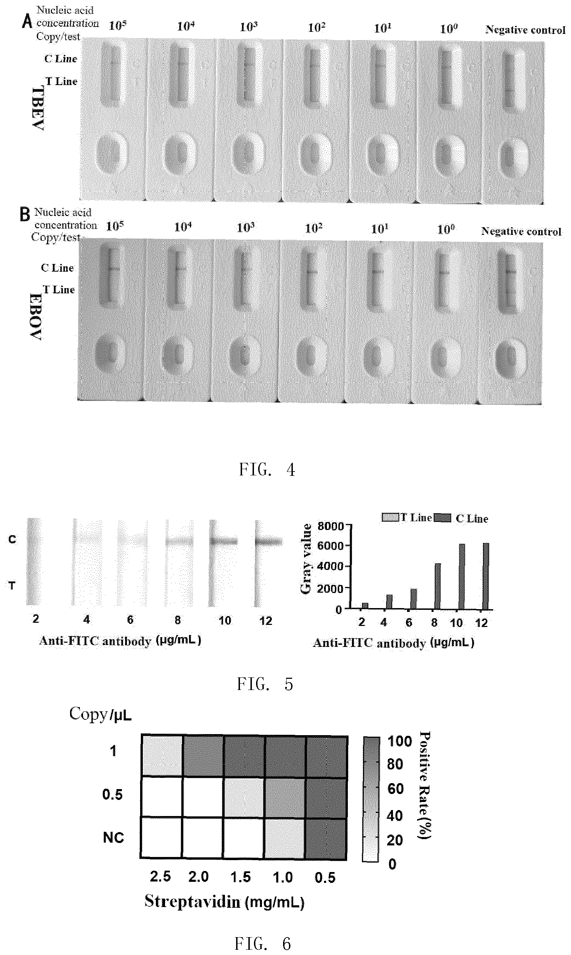

The results are shown in Fig. 4, A is Ebola virus (EBOV) nucleic acid (AF086833.2), B is forest encephalitis virus (TBEV) nucleic acid (MH645618.1). Except for the negative group shows the C line and the T line simultaneously (judged to be negative), the rest of groups only show the C line (judged to be positive).

The above results show that the sensitivity of this kit to both Ebola virus and forest encephalitis virus is single copy (10⁰ copies/µL).

### Example 7. Optimization of various components in the nucleic acid detection kit based on the CRISPR detection principle and the "line elimination" immunochromatography

### I. The optimization process of different colloidal gold-labeled rabbit derived anti-FITC antibody concentrations in strip strips

The detection method is the same as that in 2 of I in Example 1, specifically as follows:

### 1) Amplification of target nucleic acids

The amplification is the same as that in II of Example 2, and the nucleic acid in the sample to be tested and the RT-RAA amplification primers are all replaced with RNase-free H₂O;

### 2) CRISPR reaction

It is the same as that in II of Example 2;

### 3) Detection using strips

Set the concentration of the colloidal gold-labeled rabbit derived anti-FITC antibody in the "line elimination" strip in I of Example 2 to 2-12 µg/mL.

The detection is the same as that in II of Example 2.

The binding of colloidal gold (0.4 mg/mL) to the rabbit derived anti-FITC antibody is indirectly reflected by observing the depth of the C line of the "line elimination" strip (measured by Image J).

The results are shown in Fig. 5. When the concentration of the colloidal gold-labeled rabbit derived anti-FITC antibody is 2-10 µg/mL, as the concentration of the antibody increases, the C line of the "line elimination" strip becomes darker (the gray value increases); when the concentration of the colloidal gold-labeled rabbit derived anti-FITC antibody continued to increase, that is, when the concentration increases to 12 µg/mL, the color of the line C of the "line elimination" strip does not change much (the gray value increased little).

Therefore, the optimal concentration of the colloidal gold-labeled rabbit derived anti-FITC antibody is 10 µg/mL, and the corresponding coating concentration is 0.02 mg/mL.

### II. Optimization of coating concentration of streptavidin in strip strips

The detection method is the same as that in II of Example 2.

### 1) Amplification of target nucleic acids

The amplification is the same as that in Example 2, and the nucleic acids in the sample to be tested are high concentration (1 copy/µL) and low concentration (0.5 copy/µL) SARS-CoV-2 RNA (SARS-CoV-2 nucleic acid standard substance, China Institute of Metrology, GBW(E)091089).

### 2) CRISPR reaction.

It is the same as that in Example 2.

### 3) Detection using strips

Set the coating concentration of streptavidin in the "line elimination" strip in I of Example 2 to 2.5-0.5 mg/mL respectively.

The method is the same as that in II of Example 2.

Each group of experiments is repeated five times, and the results are shown in Fig. 6. At 2.5 mg/mL, the negative control group and the low concentration group are not detected at all, and the high concentration group is detected once and not detected 4 times, with a detection rate of 20%. At 2.0 mg/mL, all of the negative control group and low concentration group are not detected, and the high concentration group is detected 4 times and not detected once, with a detection rate of 80%. At 1.5 mg/mL, all of the negative control groups are not detected, detected once and not detected 4 times at low concentration group, with a detection rate of 20%, all detected in the high concentration group. At 1.0 mg/mL, detected once and not detected 4 times in the negative control group, the detection rate is 20%, 3 times detected at the low concentration group, with a detection rate of 60%, and all detected at the high concentration group. At 0.5 mg/mL, all detected in the negative control group, the low concentration group, and the high concentration group. Since the sensitivity can reach 1 copy/µL when the streptavidin coating concentration is 1.5 mg/mL, and the negative control group has a positive result (false positive) after continuing to reduce the streptavidin coating concentration, the optimum coating concentration of streptavidin is 1.5 mg/mL.

### III. Different reporter RNAs in the CRISPR reaction system

Four reporter RNAs containing different types and numbers of bases are synthesized by the method of RNA modification. The specific sequences of the reporter RNAs are 6A, AAAAAA; 5U, UUUUU; 14U, UUUUUUUUUUUUUUU; 20U, UUUUUUUUUUUUUUUUUUUUU; both ends are labeled with FAM and biotin.

The detection method is the same as that in II of Example 2.

### 1) Amplification of target nucleic acids

The amplification is the same as that in II of Example 1, and the nucleic acid in the sample to be tested is replaced with 1 copy/µL of SARS-CoV-2 RNA (that is, a positive sample, SARS-CoV-2 nucleic acid standard substance, National Institute of Metrology, GBW (E)091089).

### 2) CRISPR reaction

It is the same as that in II of Example 2, wherein the above four reporter RNAs are used respectively.

### 3) Detection using strips

The detection is the same as that in II of Example 2.

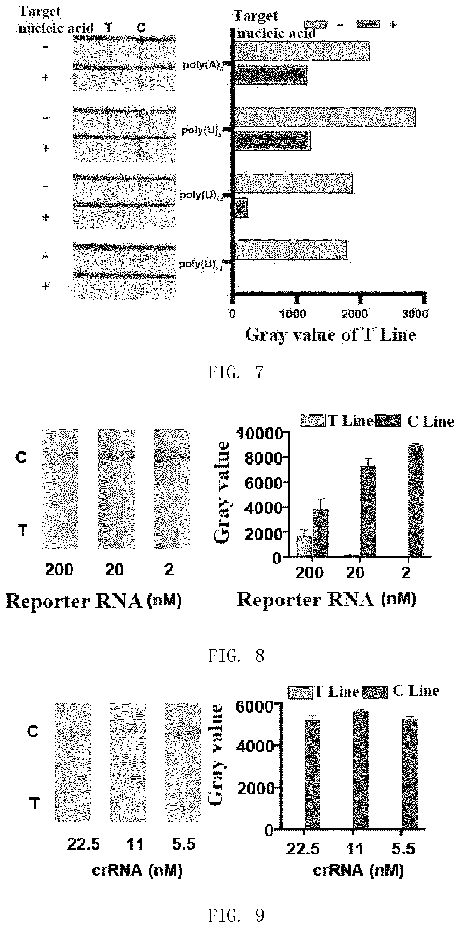

The test results are measured by Image J for the T line gray value, and the results are shown in Fig. 7. The difference between the T lines of the 5U and 6A reporter RNAs on the "line elimination" strips between the positive sample and the negative control is weak. Most of T lines of the reporter RNA for 14U disappeared in the test results of positive samples, but not completely. T lines of reporter RNAs for 20 U completely disappeared on the strip strips in the test results of positive samples. It shows that the reporter RNA for 20 U is more suitable for the "line elimination" nucleic acid detection strip disclosed in the present invention.

### IV. The concentration of each substance in the CRISPR reaction system is different

### 1. Optimization process of concentrations of different reporter RNAs

The detection method is the same as that in II of Example 2.

### 1) Amplification of target nucleic acids

The amplification is the same as that in II of Example 2, and the nucleic acid in the sample to be tested is replaced with 1 copy/pL of SARS-CoV-2 RNA (SARS-CoV-2 nucleic acid standard substance, National Institute of Metrology, GBW(E)091089).

### 2) CRISPR reaction

It is the same as that is II of Example 2.

The concentrations of reporter RNAs for 20 U are 2, 20, and 200 nM respectively.

### 3) Detection using strips

The detection is the same as that in II of Example 2.

Each group is repeated three times, and the detection results are determined by using Image J to measure the gray value of the bands. The results are shown in Fig. 8. When the final concentration of reporter RNA is 200 nM, the T line appeared obviously. When the final concentration of reporter RNA is 20 nM, the T line disappears incompletely. When the final concentration of reporter RNA is 2 nM, the T line disappears completely. Therefore, the final concentration of reporter RNA in the system is selected as 2 nM. That is, 5 µL of 20 mM reporter RNA is added to 50 µL of the system.

### 2. Selection of the optimal concentration of crRNA

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The concentrations of crRNA are 5.5, 11, and 22.5 nM respectively.

### 3) Detection using strips

The detection is the same as that in II of Example 2.

Each group is repeated three times, and the detection results are determined by using Image J to measure the gray value of the bands. The results are shown in Fig. 9. When the final concentration of crRNA is 22.5 nM, 11 nM or 5.5 nM, the T line disappeared completely. Considering that RNA is easy to degrade, the final concentration of crRNA in the system is selected as 22.5 nM. That is, 3 µL of 375 nM crRNA is added to 50 µL of the system.

### 3. Selection of optimal concentration of rNTP

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in Example 2, and the concentrations of rNTP are 0.5, 1, and 2 mM respectively.

### 3) Detection using strips

It is the same as that in II of Example 2.

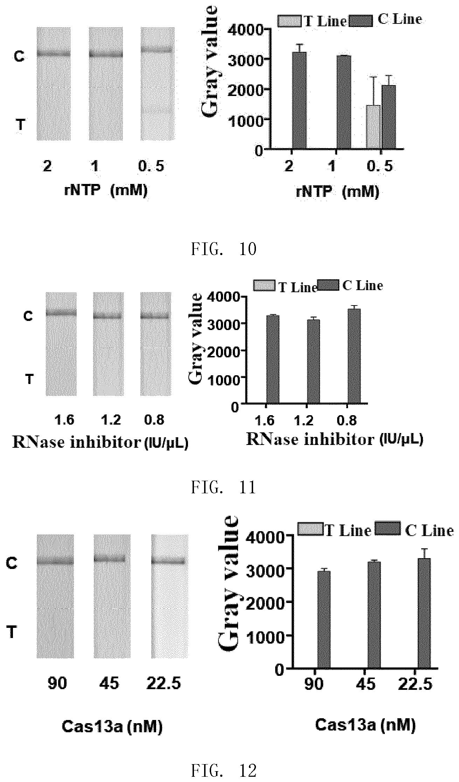

Each group is repeated three times, and the detection results are determined by using Image J to measure the gray value of the bands. The results are shown in Fig.10. When the final concentration of rNTP is 0.5 mM, the T line appeared obviously. When the final concentration of rNTP is 1 mM or 2 mM, the T line disappears completely. In order to ensure the complete reaction, the amount raw materials are appropriately increased without affecting the normal reaction. Therefore, the final concentration of rNTP in the system is selected as 2 mM. That is, 4 µL of 25 mM rNTP is added to 50 µL of the system.

### 4. Selection of the optimal concentration of RNase inhibitor

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The concentrations of RNase inhibitors are 0.8, 1.2, and 1.6 mM respectively.

### 3) Detection using strips

The detection is the same as that in II of Example 2.

Each group is repeated three times, and the detection results are determined by using Image J to measure the gray value of the bands. The results are shown in Fig. 11. When the final concentration of RNase inhibitor is 1.6 IU/µL, 1.2 IU/µL, and 0.8 IU/µL, the T line disappeared completely. In order to ensure that the reaction is not interfered by exogenous RNase, the amount of RNase inhibitor should be appropriately increased without affecting the normal reaction. Therefore, the final concentration of RNase inhibitor in the system is selected as 1.6 IU/µL. That is, 2 µL of 40 IU/µL RNase inhibitor (2 µL RNase inhibitor (Murine RNase inhibitor, NEB, M0314L)) is added into 50 µL of the system.

### 5. Selection of optimal concentration of Cas13a

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The concentration of Cas13a protein is 90nM, 45nM or 22.5nM respectively.

### 3) Detection using strips

The detection is the same as that in II of Example 2.

Each group is repeated three times, and the detection results are determined by using Image J to measure the gray value of the bands. The results are shown in Fig. 12. When the final concentration of Cas 13a protein is 90nM, 45nM or 22.5nM, the T line disappeared completely. In order to ensure the complete reaction, the amount of Cas13a protein is appropriately increased without affecting the normal reaction. Therefore, the final concentration of Cas13a protein in the system is 45.2 nM. That is, 2 µL of 1.13 µM Cas13a protein is added to 50 µL of the system.

### 6. Selection of optimal concentration of T7 transcriptase

The detection method is the same as that in II of Example 2.

### 1) Amplification of target nucleic acids

The amplification is the same as that in II of Example 2, and the nucleic acid in the sample to be tested is replaced with 1 copy/pL of SARS-CoV-2 RNA (SARS-CoV-2 nucleic acid standard substance, National Institute of Metrology, GBW(E)091089).

### 2) CRISPR reaction

It is the same as that in II of Example 2. The concentration of T7 transcriptase is 0.5 IU/µL or 1 IU/µL respectively.

### 3) Detection using strips

The detection is the same as that in II of Example 2.

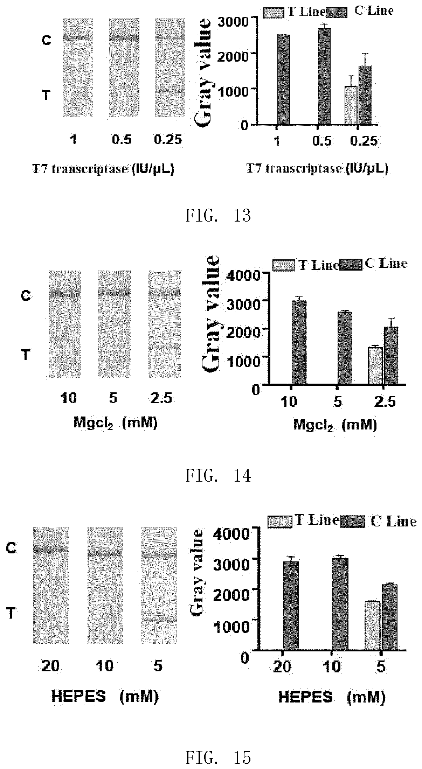

Each group is repeated three times, and the detection results are determined by using Image J to measure the gray value of the bands. The results are shown in Fig. 13. When the final concentration of T7 transcriptase is 0.25 IU/µL, the T line appeared obviously. When the final concentration of T7 transcriptase is 0.5 IU/µL or 1 IU/µL, the T line disappeared completely. In order to ensure the complete reaction, the amount of T7 transcriptase should be appropriately increased without affecting the normal reaction. Therefore, the final concentration of T7 transcriptase in the system is 1 IU/µL. That is, 1 µL of 50 IU/µL T7 transcriptase (1 µL T7 RNA polymerase (NEB, M0251S)) is added to the 50 µL system.

### 7. Selection of the optimum concentration of MgCl₂

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The concentration of MgCl₂ is 2.5 mM, 5 mM or 10 mM respectively.

### 3) Detection using strips

The detection is the same as that in II of Example 2.

Each group is repeated three times, and the detection results are determined by using Image J to measure the gray value of the bands. The results are shown in Fig. 14. When the final concentration of MgCl₂ is 2.5 mM, the T line appeared obviously. When the final concentration of MgCl₂ is 5 mM or 10 mM, the T line disappears completely. In order to ensure the complete reaction, the amount of catalyst is appropriately increased without affecting the normal reaction. Therefore, the final concentration of MgCl₂ in the system is selected as 10 mM. That is, 0.5 µL of 1 M MgCl₂ is added to 50 µL of the system.

### 8. Selection of optimum concentration of HEPES

The detection method is the same as that in II of Example 2.

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The final concentration of HEPES is 5mM, 10mM or 20mM respectively.

### 3) Detection using strips

The detection is the same as that in II of Example 2.

Each group is repeated three times, and the detection results are determined by using Image J to measure the gray value of the bands. The results are shown in Fig. 15. When the final concentration of HEPES is 5 mM, the T line appeared obviously. When the final concentration of HEPES is 10 mM or 20 mM, the T line disappears completely. In order to ensure the stability of the reaction system, the amount of buffer is appropriately increased without affecting the normal reaction. Therefore, the final concentration of HEPES in the system is selected as 20 mM. That is, 1 µL of 1 M MgCl₂ is added to 50 µL of the system.

### 9. Selection of optimal loading volume of nucleic acids

### 1) Amplification of target nucleic acids

The amplification is the same as that in II of Example 2, and the nucleic acid in the sample to be tested is replaced with 1 copy/µL of SARS-CoV-2 RNA (SARS-CoV-2 nucleic acid standard substance, National Institute of Metrology, GBW(E)091089).

### 2) CRISPR reaction

It is the same as that in II of Example 2. The amount of RT-RAA product is 2, 5 or µL respectively;

### 3) Detection using strips

The detection is the same as that in II of Example 2.

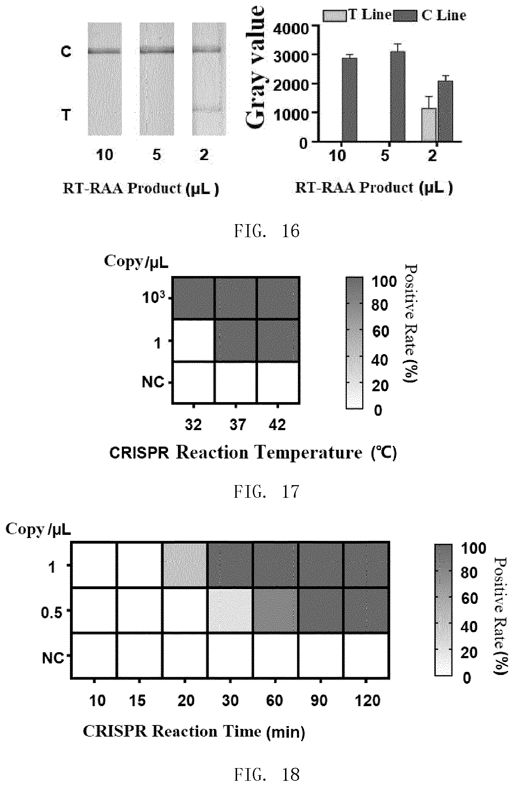

Each group is repeated three times, and the detection results are measured by Image J for the gray value of the bands. The results are shown in Fig. 16. When the amount of RT-RAA product is 2 µL, the T line appeared obviously. When the amount of RT-RAA product is 5 µL or 10 µL, the T line completely disappeared. Therefore, in order to ensure the normal reaction, at least 5 µL of RT-RAA product is added. That is, the amount of RT-RAA product in the system is 5 µL.

### 10. Screening of the optimum temperature for CRISPR detection

### 1) Amplification of target nucleic acids

The amplification is the same as in II of Example 2, and the nucleic acid in the sample to be tested is replaced with high concentration (10³ copies/µL) and low concentration (1 copy/µL) SARS-CoV-2 RNA (SARS-CoV-2 nucleic acid standard substance, China Institute of Metrology, GBW(E)091089).

### 2) CRISPR reaction

It is the same as that in II of Example 2. The temperature for CRISPR reaction is 32°C, 37°C and 42°C respectively.

### 3) Detection using strips

The detection is the same as in II of Example 2.

Each group of experiments is repeated five times, and the results are shown in Fig. 17. At 32°C, the negative control group and the low concentration group are all undetected, and the high concentration groups are all detected. At 37°C and 42°C, all the negative control groups are not detected, and low concentration group and high concentration group are all detected. Since 37°C is closer to room temperature, and this temperature is easier to achieve, so the optimal reaction temperature for CRISPR detection is 37°C.

### 11. Selection of optimal CRISPR reaction time

### 1) Amplification of target nucleic acids

The amplification is the same as that in 2 of II of Example 1, but the nucleic acid in the sample to be tested is replaced with high concentration (1 copy/µL) and low concentration (0.5 copy/µL) SARS-CoV-2 RNA (SARS-CoV-2 nucleic acid standard substance, China Institute of Metrology, GBW(E)091089).

### 2) CRISPR reaction

It is the same as that in II of Example 2. The CRISPR reaction time is 10-120 min.

### 3) Detection using strips

The detection is the same as that in II of Example 2.

Each group of experiments is repeated five times, and the results are shown in Fig.18. At 10 min and 15 min, all groups are not detected. At 20 min, all the negative control group and low concentration group are not detected, and the high concentration groups are detected 2 times, not detected 3 times, the detection rate is 40%. At 30 min, all the negative control groups are not detected, the low concentration group is detected 1 time, not detected 4 times, the detection rate is 20%, and all the high concentration groups are detected. At 60 min, none of the negative control groups are detected, the low concentration groups are detected 4 times, and not detected 1 time, the detection rate is 80%, and all the high concentration groups are detected. At 90 min and 120 min, none of the negative control groups are detected, and all the low concentration group and high concentration group are detected. Since the sensitivity at 30 min can be 1 copy/µL, and the sensitivity of 0.5 copies/µL needs to be reacted for 90 min, according to the positioning of on-site rapid detection technology, the optimal reaction time of CRISPR detection is 30 min.

### 12. Selection of the most suitable time for "line elimination" strip chromatography

### 1) Amplification of target nucleic acids

The amplification is the same as that in II of Example 2, and the nucleic acid in the sample to be tested is replaced with high concentration (10³ copies/µL) and low concentration (1 copy/µL) SARS-CoV-2 RNA (SARS-CoV-2 nucleic acid standard substance, China Institute of Metrology, GBW(E)091089).

### 2) CRISPR reaction

It is the same as that in II of Example 2.

### 3) Detection using strips

The detection is the same as in II of Example 2. The readout time of the strip results are 2 min, 5 min, 15 min and 30 min respectively.

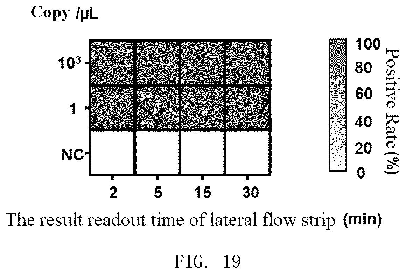

Each group of experiments is repeated five times, and the results are shown in Fig.19. At 2 min, 5 min, 15 min and 30 min, none of the negative control groups is detected, but all the low concentration group and high concentration group are detected. Therefore, the optimum readout time of strip results is 2 min.

As can be seen from the above, the optimum reaction system and reaction conditions are the conditions in Example 1.

### Example 8. Exploration of primers used to detect 2019-nCoV in nucleic acid detection kits

### I. Design the following primers

Design the following primers for RT-RAA amplification of 2019-nCoV
2019-nCoV-F-N (Forward primer): aattctaatacgactcactatagggAATTGCACAATTTGCCCCCAGCGCTTCAG (SEQ ID NO: 11);
2019-nCoV-R-N (Reverse primer): CTTGATCTTTGAAATTTGGATCTTTGTCAT (SEQ ID NO: 12);

### II. Detection

### 1) Amplification of target nucleic acids

100, 10, 1, 0.5, 0.1 copies/pL of the SARS-CoV-2 standard material (National Institute of Metrology, GBW(E)091089) as a template is amplified using the primer pair 2019-nCoV-F-N/2019-nCoV-R-N the primer pair 2019-nCoV-F1/2019-nCoV-R1 respectively.

The RT-RAA amplification kit is used to pre-amplify the target nucleic acids. The steps are as follows: firstly, prepare a mixture of 30 µL template, 2 µL upstream and downstream primers (10 µM), and 17.5 µL A Buffer, and add it to a container containing lyophilized powder. In the basic reaction unit, the lyophilized powder is fully redissolved evenly. Add 2.5µL B Buffer solution to the cap of each reaction tube, close the cap, invert it up and down 5-6 times to mix well, and centrifuge at a high speed for 10 seconds. The above reaction tube is placed at 42°C for 30 min to obtain RT-RAA amplification products of different primer pairs. The remaining methods and steps are the same as in Example 2, and a negative control group (H₂O) is prepared, and each group is repeated 10 times.

### 2) CRISPR reaction

The method is the same as that in II of Example 2, except that the RT-RAA amplification products amplified by different primers in 1) above are used.

The sensitivity comparison results of the 2019-nCoV-F-N/2019-nCoV-R-N primer pair (also known as the new primer) and the 2019-nCoV-F1/2019-nCoV-R1 primer pair (also known as the original primer) are shown in Table 2:

**Table 2 is the statistical table of primer pair comparison results**

| Template (copies/µL) | 2019-nCoV-F-N/2019-nCoV-R-N Primer | | | 2019-nCoV-F1/2019-nCoV-R1 Primer | | |
|---|---|---|---|---|---|---|
| | Positive (time) | Negative (time) | Positive rate (%) | Positive (time) | Negative (time) | Positive rate (%) |
| 100 | 10 | 0 | 100 | 10 | 0 | 100 |
| 10 | 10 | 0 | 100 | 10 | 0 | 100 |
| 1 | 10 | 0 | 100 | 10 | 0 | 100 |
| 0.5 | 10 | 0 | 100 | 4 | 6 | 40 |
| 0.1 | 6 | 4 | 60 | 0 | 10 | 0 |
| Negative control | 0 | 10 | 0 | 0 | 10 | 0 |

It can be seen that when the template concentration is greater than 1 copies/µL, both the new primers and the original primers can detect. That is, the detection rate is 100%. When the template concentration is 0.5 copies/µL, all the new primers can detect. That is, the detection rate is 100%. The original primer detects 4 times. That is, the detection rate is 40%. When the template concentration is 0.1 copies/pL, the new primer detects 6 times. That is, the detection rate is 60%. The original primer detects 0 time. That is, the detection rate is 0%. Neither the new primer nor the original primer detects in the negative control group. It can be seen from the above results that the sensitivity of the new primer is higher than that of the original primer.

Therefore, when detecting 2019-nCoV, the primer pairs in the optimal reaction system and reaction conditions in Example 1 can be replaced.

The above are just preferred embodiments of the present invention, it should be pointed out that some improvements and modifications can also be made for those of ordinary skill in the art without departing from the technical principle of the present invention. These improvements and modifications should also be considered as falling within the protection scopes of the present invention.

### Industrial application

The present invention provides a lateral flow strip based on the "line elimination and a target nucleic acid detection kit with matching reporter RNA. The strip in the kit uses the complete disappearance of the "T" line as the judgment standard for positive results (that is, the occurrence of 1 line is positive, and two lines are negative). Under the premise of ensuring the sensitivity of CRISPR nucleic acid detection, false positive results caused by subjective interpretation errors are avoided, and the accuracy of CRISPR nucleic acid detection results is improved. CRISPR nucleic acid detection system can be combined to realize high sensitivity, high specificity and convenient detection for various specific nucleic acids (pathogens, gene mutations, drug resistance mutations, etc.).

## Claims

1. A target nucleic acid detection kit, comprising the following:
1) immunochromatographic strip
the immunochromatographic strip comprises a sample pad containing a colloidal gold-labeled antibody, an NC membrane containing a T line and a C line, and a absorbent pad in sequence in the flow direction of the sample;
the T line is coated with streptavidin;
the C line is coated with a secondary antibody of the colloidal gold-labeled antibody;
2) CRISPR reaction system
the CRISPR reaction system includes a reporter RNA, a crRNA and a Cas protein;
the Cas protein is the Cas protein in the class II type V or VI CRISPR system;
both ends of the reporter RNA are respectively labeled with biotin and a group capable of binding to the colloidal gold-labeled antibody;
the crRNA includes a region binding to Cas protein and a region binding to a target nucleic acid in a target sequence.

2. The kit according to claim 1, **characterized in that**:
the Cas protein is *Lw*Cas13a protein, Cas12a or Cas12b protein.

3. The kit according to claim 1 or 2, **characterized in that**:
the reporter RNA consists of 20 U, and both ends of the reporter RNA are labeled with biotin and fluorescein respectively.

4. The kit according to any one of claims 1-3, **characterized in that**:
the kit further comprises reagents for amplifying the target nucleic acid.

5. The kit according to claim 4, **characterized in that**:
the amplification is RAA isothermal amplification, PCR variable temperature amplification or LAMP isothermal amplification.

6. The kit according to any one of claims 1-5, **characterized in that**:
the target nucleic acid is a pathogenic nucleic acid, a nucleic acid with a single nucleotide polymorphism or a drug resistant nucleic acid.

7. The kit according to claim 6, **characterized in that**:
the pathogen nucleic acid is from SARS-CoV-2, Ebola virus or forest encephalitis virus.

8. The kit according to any one of claims 1-7, **characterized in that**:
the colloidal gold-labeled antibody is a colloidal gold-labeled anti-FITC antibody.

9. The kit according to claim 8, **characterized in that**:
the components of the CRISPR reaction system include: the reporter RNA, the crRNA, LwCas13a protein, NTP Mix, T7 RNA polymerase, RNase inhibitor, MgCl₂, HEPES buffer;
the coating concentration of the colloidal gold-labeled anti-FITC antibody is 0.016-0.16 mg/mL;
the coating concentration of the streptavidin is 1.0-2.0 mg/mL;
the concentration of the reporter RNA contained in the CRISPR reaction system is 2-20 nM;
the concentration of the crRNA in the CRISPR reaction system is 5.5-55 nM;
the concentration of the rNTP contained in the CRISPR reaction system is 1-10 mM;
the concentration of an RNase inhibitor in the CRISPR reaction system is 0.8-8 IU/µL;
the concentration of Cas13a protein contained in the CRISPR reaction system is 22.5-225 nM;
the concentration of T7 transcriptase contained in the CRISPR reaction system is 0.5-5 IU/µL;
the concentration of MgCl₂ contained in the CRISPR reaction system is 5-50 mM;
the concentration of HEPES contained in the CRISPR reaction system is 10-100 mM; and
the amount of RT-RAA product in the CRISPR reaction system is 5-10 µL.

10. The kit according to any one of claims 1-9, **characterized in that**:
if the target nucleic acid to be detected is a SARS-CoV-2 genome, the nucleotide sequence of the corresponding crRNA is SEQ ID NO: 2, and the primers in the RT-RAA amplification kit for amplifying the target nucleic acid are a primer shown in SEQ ID NO: 3 or SEQ ID NO: 11 and a primer shown in SEQ ID NO: 4 or SEQ ID NO: 12;
if the target nucleic acid to be detected is EBOV genome, the nucleotide sequence of the corresponding crRNA is SEQ ID NO: 5, and the primers in the RT-RAA amplification kit for amplifying the target nucleic acid are EBOV-F1 primer shown in SEQ ID NO: 6 and EBOV-R1 primer shown in SEQ ID NO: 7;
if the target nucleic acid to be detected is TBEV genome, the nucleotide sequence of the corresponding crRNA is SEQ ID NO: 8, and the primers in the RT-RAA amplification kit for amplifying the target nucleic acid are TBEV-F1 primer shown in SEQ ID NO:9 and TBEV-R1 primer shown in SEQ ID NO: 10.

11. Use of a kit according to any one of claims 1-10 in manufacturing a product having at least one of the following functions in 1)-4):
1) detect whether a target nucleic acid is a pathogenic nucleic acid;
2) detect a SNP site polymorphism of a target nucleic acid;
3) detect whether a target nucleic acid is a drug resistance gene; and
4) detect whether a sample to be tested contains a pathogenic nucleic acid.

12. The use according to claim 11, **characterized in that**:
the nucleic acid of the pathogen is from SARS-CoV-2, Ebola virus or forest encephalitis virus.

13. A method for detecting whether a target nucleic acid is a pathogenic nucleic acid, comprising the steps of:
1) amplify a target nucleic acid using a reagent for isothermally amplifying RAA in the kit according to any one of claims 1-10 to obtain an RT-RAA amplification product;
2) add the RT-RAA amplification product to the CRISPR reaction system in the kit according to any one of claims 1-10 to perform a CRISPR reaction to obtain a CRISPR reaction product;
3) detect the CRISPR reaction product using the immunochromatographic strip in the kit according to any one of claims 1-10,
if the strip has no T line and a C line appears, the target nucleic acid is a pathogenic nucleic acid or a candidate for a pathogenic nucleic acid, and it is judged to be positive. If the strip has a T line and a C line, the target nucleic acid is not a target nucleic acid or a candidate for a target nucleic acid, and it is judged to be negative.

14. A method for detecting whether a sample to be tested is a nucleic acid of a pathogen, comprising the following steps:
1) amplify a sample to be tested using the reagent for isothermally amplifying RAA in the kit according to any one of claims 1-10 to obtain RT-RAA amplification products;
2) add the RT-RAA amplification products to the CRISPR reaction system in the kit according to any one of claims 1-10 to perform a CRISPR reaction to obtain CRISPR reaction products;
3) detect the CRISPR reaction product using the immunochromatographic strip in the kit according to any one of claims 1-10,
if the strip has no T line and a C line appears, the sample to be tested contains a pathogenic nucleic acid or a candidate for the pathogenic nucleic acid, and it is judged to be positive. If the strip has a T line and a C line, the sample to be tested does not contain the target nucleic acid or a candidate for the target nucleic acid, and is judged to be negative.

15. The method according to claim 13 or 14, **characterized in that**:
the nucleic acid of the pathogen is from SARS-CoV-2, Ebola virus or forest encephalitis virus.
